# EUROPEAN PATENT APPLICATION

(11) **EP 4 537 858 A1**
(43) Date of publication of application: **16.04.2025**
(21) Application number: 23202418.2
(22) Date of filing: 09.10.2023
(51) Int. Cl.: A61L 9/12, A01M 1/20

(54) **DEVICE FOR DIFFUSING VOLATILE SUBSTANCES**

(71) Applicant: Zobele Holding SpA, 38100 Trento (IT)
(72) Inventor: YAO SI JING, Charles, GUANGDONG, 518125 (CN); DECOBERT, James Eugene, Guangdong, 518125 (CN)
(74) Representative: Herrero & Asociados, S.L.

(57) **Abstract**

The device for diffusing volatile substances comprises a container (1) containing a liquid with the volatile substances; a wick (2) partially placed inside the container (1) in contact with the liquid, said wick (2) having at least a portion with a non-cylindrical shape; a stopper (5); and a cap (4) that is threaded on the stopper (5), said cap being removed for using the device, wherein the device for diffusing volatile substances also comprises a cover (3) that covers the wick (2), and the stopper (5) comprises an antirotation element (51) for preventing the rotation of the cover (3) with respect to the stopper (5).

It permits to provide a device for diffusing volatile substances wherein the wick is not cylindrical, but the cap is threaded.

## Description

The present invention relates to a device for diffusing volatile substances.

### Background of the invention

Devices for diffusing volatile substances, such as air fresheners or mosquito repellent devices, are known for years.

Usually, in these devices, a liquid is contained inside a container and comprises a porous element with capillary properties, i.e., a wick. This wick transports the liquid from the inside of the container to the outside of a container.

Then, from the outside of the container the liquid can be evaporated directly from the wick, with or without the support of some extra features to increase volatility, such as heat and/or air flow.

In other examples, the liquid is transferred from the top side of the wick to another element that contributes to the diffusion of the volatile substance in the atmosphere, such as another porous element, or an ultrasonic nebulizer or any other known technology.

The wick usually has a cylindrical geometry, and it is inserted in a neck of the container, sometime with the help of a stopper that makes the union between the wick and the bottle neck, and the container is closed by a cap that is screwed on the neck.

The need of having a cylindrical wick comes from the necessity to screw the cap, i.e., to rotate it around the longitudinal axis, while having minimal headspace between the wick and the cap.

Any headspace, i.e., an empty volume, between the wick and the cap, is a space where volatile substance could accumulate during transportation and storage and could lead to leakage when cap is removed.

On the other side, cylindrical wicks, although widely used, are maybe not the best support for evaporation. In fact, an important aspect is the surface, as evaporation is the combination of volatility of the substance plus the amount of surface exposed to the atmosphere, so that the cylindrical shape of the wick is rather an issue than an advantage.

In addition, when volatility is increased due to a heater, the heater is generally having a singular geometry and it is always a challenge to adapt its heat to a cylindrical geometry evaporation surface.

So, apart from the necessity to screw a cap on top of the wick, there are several reasons to argue than a flat wick is better than a cylindrical wick, but there is a need to solve how to close the bottle with a cap.

Furtherly, screw caps are closures that are quite intuitive for the user, while other systems may not be.

### Description of the invention

Therefore, an objective of the present invention is to provide a device for diffusing volatile substances wherein the wick is not cylindrical, but the cap is threaded.

With the device for diffusing volatile substances of the invention, the aforementioned drawbacks are resolved, presenting other advantages that will be described below.

The device for diffusing volatile substances according to the present invention is defined in claim 1. Additional optional features are included in the dependent claims.

In particular, the device for diffusing volatile substances comprises:
- a container containing a liquid with the volatile substances;
- a wick partially placed inside the container in contact with the liquid, said wick having at least a portion with a non-cylindrical shape;
- a stopper; and
- a cap that is threaded on the stopper, said cap being removed for using the device,
- a cover that covers the wick,
wherein the stopper also comprises an anti-rotation element, such as a flange or recess, for preventing the rotation of the cover with respect to the stopper.

Furthermore, the cover is connected to the cap, permitting the rotation of the cap with respect to the cover, so that when the cap is removed, the cover also removed.

Advantageously, the shape of the cover corresponds to the shape of the wick.

According to a preferred embodiment, the wick is flat.

Furthermore, the cover preferably comprises a protrusion inserted inside a hole of the cap, and this protrusion comprises a rim.

According to a preferred embodiment, the cover comprises a base and a housing for housing the wick.

Preferably, the base is joined to the housing by inclined walls, and the base comprises ribs.

According to a preferred embodiment, the housing of the cover has a paralelepipedic shape.

Furthermore, the stopper also comprises an anti-rotation element, such as a flange or recess, for preventing the rotation of the cover with respect to the stopper.

Furthermore, the inclination of the inclined walls is the same than the inclination of the anti-rotation element.

For its threaded connection, the cap comprises a skirt that is internally threaded and the stopper comprises an external thread.

Furthermore, the cover can comprise preferably a perimetral rib in contact with the stopper, that coincides with the area where the cap generates high stress on the cover when screwed on the bottle neck. This high stress perimetral area will improve the features against leakage of the device.

Preferably, the stopper comprises a vent hole inside which a protrusion of the cover is housed, and a centering hole placed opposite to the vent hole, inside which a protrusion of the cover is housed.

### Brief description of the drawings

For a better understanding of what has been stated, some drawings are attached in which, schematically and only as a non-limiting example, a practical case of embodiment is represented.

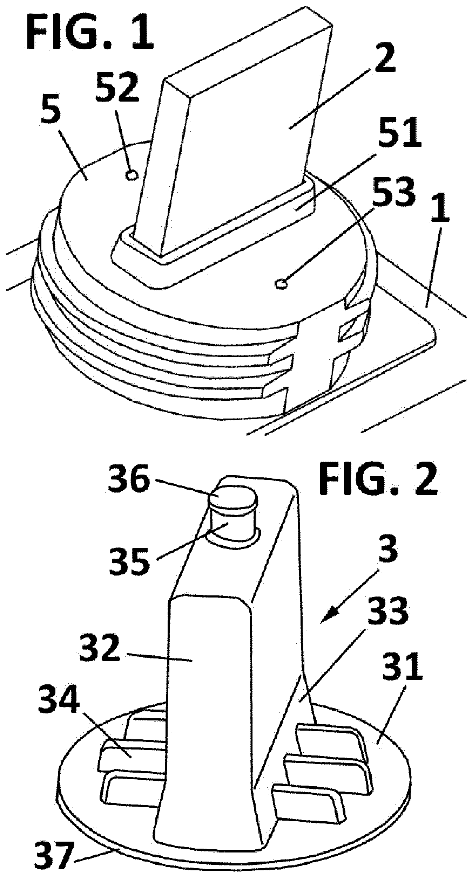

Figure 1 is a perspective view of the wick of the device for diffusing volatile substances according to the present invention inserted in the container;
Figure 2 is a perspective view of the cover for the wick of the device for diffusing volatile substances according to the present invention;

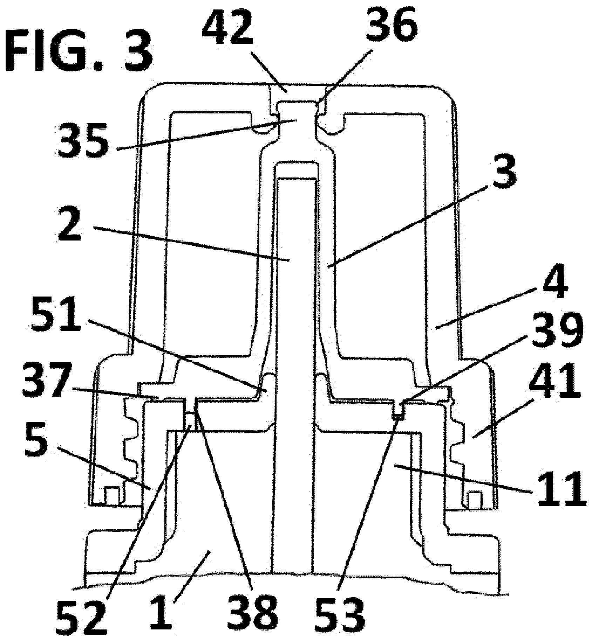

Figure 3 is an elevation sectional view of the device for diffusing volatile substances according to the present invention.

### Description of a preferred embodiment

The device for diffusing volatile substances according to the present invention comprises a container 1 comprising an aperture or neck 11 and containing a liquid with the volatile substances and a wick 2 that is partially placed inside the container 1 in contact with the liquid.

At least a portion of said wick 2 has a non-cylindrical shape, such as comprising at least a flat surface, e.g., is a flat wick.

It must be pointed out that in the present description and in the claims non-cylindrical wick means that the cross-section of at least a portion of the wick is not circular. In particular, examples of a non-cylindrical wick are a wick with a polygonal cross-section, such a square or rectangle, a wick with a cross-section defining an oval, etc.

Cross-section means the section obtained by a plane perpendicular to a longitudinal axis of the wick.

The device for diffusing volatile substances also comprises a cover 3 that covers the wick 2, a cap 4 placed on the cover 3, and a stopper 5, that is placed on the neck 11 and closes the container 1. Both the cover 3 and the cap 4 are removed before a first use of the device.

The cover 3 comprises a base 31 and a housing 32 that are connected to each other by inclined walls 33. The base 31 contacts the container 1 when the container 1 is closed, and the housing 32 has a shape that corresponds to the shape of the wick 2.

According to the shown embodiment, the cover 3 also comprises ribs 34 that are designed to reduce a possible deformation of the base 31 when screwed on the container 1, to avoid the creation of gaps that can be filled by the liquid during storage.

According to the shown embodiment, the wick 2 is flat, and the housing 32 has a paralelepipedic shape.

The cap 4 has a cylindrical geometry and comprises a skirt 41 that is internally threaded.

This skirt 41 engages with the external part of the stopper 5, that is externally threaded, so that the cap 4 and the stopper 5 are threaded to each other.

Furthermore, the stopper 5 comprises an anti-rotation element, such as a flange 51, that contacts the wick 2, and it is inclined in correspondence with the inclined walls 33 of the cover 3.

This flange 51 holds the wick 2 in the correct position, guides the cover 3 when it is introduced on the wick 2, and avoids the rotation of the cover 3 with respect to the stopper 5, when the cap 4 is rotated.

It must be pointed out that the flange 51 can be substituted by a recess, not shown in the drawings, complementary with a flange of the cover 3, to avoid also the rotation of the cover 3.

Furthermore, the lower face of the cover 3 comprises a perimetral rib 37 in contact with the stopper 5, 6 that coincides with the area where the cap 4 generates high stress on the cover 3 when screwed on the bottle neck 11. This high stress perimetral area will improve the features against leakage of the device.

Furthermore, the stopper 5 comprises a vent hole 52, that is a through-hole, inside which a protrusion 38 of the cover 3 is housed, and a centering hole 53 placed opposite to the vent hole 52, inside which a protrusion 39 of the cover 3 is housed.

The cover 3 and the cap 4 are joined together through a protrusion 35 placed on top of the cover 3 and that is clipped to the cap 4, but still allowing rotational movement between the cover 3 and the cap 4. In particular, the protrusion 35 is cylindrical and it is placed inside a hole 42 of the cap 4, the cylindrical portion 35 having a rim for preventing the separation of the cap 4 from the cover 3. This way, when a user removes the cap 4, the cover 3 can also be removed with the same movement.

The mechanical clipping also allows easy manufacturing, including automatic manufacturing, where first the cover 3 is placed oriented with respect to the wick 2, and then the cap 4 is placed on the cover 3, clipped and then screwed on the container 1.

Despite the fact that reference has been made to a specific embodiment of the invention, it is clear to a person skilled in the art that the described device for diffusing volatile substances is susceptible to numerous variations and modifications, and that all the mentioned details can be substituted by others technically equivalent ones, without departing from the scope of protection defined by the appended claims.

## Claims

1. Device for diffusing volatile substances, comprising:
- a container (1) containing a liquid with the volatile substances;
- a wick (2) partially placed inside the container (1) in contact with the liquid, said wick (2) having at least a portion with a non-cylindrical shape;
- a stopper (5); and
- a cap (4) that is threaded on the stopper (5), said cap being removed for using the device,
**characterized in that** the device for diffusing volatile substances also comprises a cover (3) that covers the wick (2), and the stopper (5) comprises an anti-rotation element (51) for preventing the rotation of the cover (3) with respect to the stopper (5).

2. Device for diffusing volatile substances according to claim 1, wherein the cover (3) is connected to the cap (4), permitting the rotation of the cap (4) with respect to the cover (3), so that when the cap (4) is removed, the cover (3) is also removed.

3. Device for diffusing volatile substances according to claim 1 or 2, wherein the shape of the cover (3) corresponds to the shape of the wick (2).

4. Device for diffusing volatile substances according to claim 3, wherein the wick (2) is flat.

5. Device for diffusing volatile substances according to any one of the previous claims, wherein the cover (3) comprises a protrusion (35) inserted inside a hole (42) of the cap (4).

6. Device for diffusing volatile substances according to claim 5, wherein the protrusion (35) comprises a rim (36).

7. Device for diffusing volatile substances according to any one of the previous claims, wherein the cover (3) comprises a base (31) and a housing (32) for housing the wick (2).

8. Device for diffusing volatile substances according to claim 7, wherein the base (31) is joined to the housing (32) by inclined walls (33).

9. Device for diffusing volatile substances according to claim 7 or 8, wherein the base (31) comprises ribs (34).

10. Device for diffusing volatile substances according to claims 4 and 7, wherein the housing (32) of the cover (3) has a paralelepipedic shape.

11. Device for diffusing volatile substances according to any one of the previous claims 1, wherein the anti-rotation element (51) is a flange or a recess.

12. Device for diffusing volatile substances according to any one of the previous claims, wherein the cap (4) comprises a skirt (41) that is internally threaded and the stopper (5) comprises an external thread.

13. Device for diffusing volatile substances according to any one of the previous claims, wherein the cover (3) comprises a perimetral rib (37) in contact with the stopper (5).

14. Device for diffusing volatile substances according to any one of the previous claims, wherein the stopper (5) comprises a vent hole (52) inside which a protrusion (38) of the cover (3) is housed.

15. Device for diffusing volatile substances according to claim 14, wherein the stopper (5) also comprises a centering hole (53) placed opposite to the vent hole (52), inside which a protrusion (39) of the cover (3) is housed.
